# EUROPÄISCHE PATENTSCHRIFT

(11) **EP 4 157 208 B1**
(45) Veröffentlichungstag und Bekanntmachung des Hinweises auf die Patenterteilung: **04.03.2026**
(21) Anmeldenummer: 21723697.5
(22) Anmeldetag: 30.04.2021
(51) Int. Cl.: A61K 8/37, A61K 8/92, A61Q 19/00, A61K 8/06

(54) **MINERALÖLFREIE W/O-EMULSION MIT VARIALBER KONSISTENZ**
MINERAL-OIL-FREE W/O EMULSION WITH A VARIABLE CONSISTENCY
ÉMULSION EAU/HUILE EXEMPTE D'HUILE MINÉRALE DE CONSISTANCE VARIABLE

(30) Priorität: 27.05.2020 DE 102020003169
(43) Veröffentlichungstag der Anmeldung: 05.04.2023
(73) Patentinhaber: Beiersdorf AG, 22529 Hamburg (DE)
(72) Erfinder: RATHSACK, Jessica, 21614 Buxtehude (DE); HARTWIG, Tanja, 23843 Bad Oldeslohe (DE); FIEDLER, Dorothe, 22335 Hamburg (DE)
(74) Vertreter: Beiersdorf AG
(86) Internationale Anmeldenummer: PCT/EP2021/061399
(87) Internationale Veröffentlichungsnummer: WO 2021/239384

(56) Entgegenhaltungen:
- WO-A1-2019/182198
- DE-A1- 102018 217 130
- KR-A- 20190 057 660
- US-A1- 2015 011 654
- DATABASE GNPD [online] MINTEL; 8 January 2019 (2019-01-08), ANONYMOUS: "Soothing Lip Balm", XP055831519, retrieved from https://www.gnpd.com/sinatra/recordpage/6245877/ Database accession no. 6245877

## Beschreibung

Die vorliegende Erfindung betrifft eine kosmetische Wasser-in-ÖI-Emulsion (W/O-Emulsion) enthaltend einen ersten Emulgator aus der Gruppe der Verbindungen Diisostearoyl Polyglyceryl-3 Dimer Dilinoleate und/oder Polyglyceryl-3 Polyricinoleate, einen zweiten Emulgator aus der Gruppe der Verbindungen Polyglyceryl-4 Diisostearate/Polyhydroxystearate/Sebacate und/oder Polyglyceryl-2 Sesquioleate sowie Sonnenblumenwachs, wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen, Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, sowie dessen Herstellungsverfahren.

Die Zubereitung kann insbesondere als Lippenpflegezubereitung (Lippenstift, Lippencreme) verwendet werden.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Neben Gesichtspudern und Rouge als pulverförmigen Kosmetika sowie stiftförmigen Zubereitungen werden hierzu cremeförmige Präparate, wie Tagescremes und Creme-Make-up auf Emulsionsbasis verwendet.

Zu den am meisten verwendeten dekorativen Kosmetika gehört der Lippenstift. Mehr als 50 % der Frauen in Deutschland benutzen ihn. Meist dient er neben der farblichen Betonung der Lippen auch zur Lippenpflege.

Lippenstifte bestehen in der Regel aus einer Wachsmatrix, in die in geringer Konzentration flüssige und halbfeste Öle sowie Pigmente und Füllstoffe eingearbeitet sind. Sie liegen in fester Stiftform vor.

Nachteilig am Stande der Technik ist der Umstand, dass Lippenpflegeprodukte wie Lippencremes, Lippenbutter etc. und insbesondere die Lippenstifte des Standes der Technik häufig Mineralöle und Paraffinwachse enthalten, die bei den Verbrauchern zunehmend unbeliebt sind. Neben einem generellen Wunsch nach Produkten aus nachwachsenden Rohstoffen, spielt dabei auch die Sorge vor gesundheitsgefährdenden Verunreinigungen in den auf Rohölbasis hergestellte Rohstoffen enthalten sein könnten eine Rolle. Ob diese Sorge tatsächlich berechtigt ist, kann im Rahmen der vorliegenden Offenbarung dahingestellt bleiben. Tatsache ist, dass bei vielen Verbrauchern der Wunsch nach "mineralölfreien" Produkten besteht. Es war daher die Aufgabe der vorliegenden Erfindung, ein "mineralölfreies" Lippenpflegeprodukt zu entwickeln.

Für die Herstellung insbesondere von Lippenstiften hat der Verzicht auf Mineralöle und Paraffinwachse jedoch gravierende Nachteile. Bei der Herstellung kommt es beim Abkühlungsvorgang der geschmolzenen Stiftmasse leicht zu optischen Mängeln, Rissen und Brüchen in der Stiftmasse.

Es war daher die Aufgabe der vorliegenden Erfindung, die Nachteile des Standes der Technik zu vermeiden und einen Lippenstift zu entwickeln, das frei ist von Mineralölprodukten und sich in hoher Qualität herstellen lässt.

Insbesondere mineralölfreie Lippenpflegeprodukte (insbesondere Lippenstifte) des Standes der Technik haben darüber hinaus den Nachteil, dass sie nicht besonders lagerstabil sind, sondern relativ stark temperatur- und lichtanfällig sind, d.h. bei Temperaturschwankungen oder Licht relativ leicht inhomogen und brüchig/rissig werden. Mineralölfreie Stifte des Standes der Technik haben darüber hinaus den Nachteil, dass sie im Vergleich zu mineralölhaltigen Stiften auf der Lippe ein schlechteres Gleitvermögen zeigen. Diese Stifte fühlen sich wachsartig/klebrig/unangenehm an.

Es war daher die Aufgabe der vorliegenden Erfindung auch diese Mängel des Standes der Technik zu reduzieren.

Nicht zuletzt stellt es ein grundsätzliches Problem bei der Entwicklung von Lippenpflegeprodukten dar, dass für jede Zubereitungsform (Stift, Creme etc.) eigene Formeln unterschiedlicher Zusammensetzung entwickelt werden müssen. Dies bedeutet nicht allein einen zusätzlichen Aufwand bei der Entwicklung und Herstellung der Produkte. Darüber hinaus haben die Anwender das Problem, dass, wenn sie mit einem Produkt, beispielsweise einem Lippenstift, sehr zufrieden sind, beim Wechsel der Produktform beispielsweise hin zu einer Creme, kein Produkt mit gleichem Leistungsspektrum (beispielsweise im Hinblick auf die Verträglichkeit, Hautbefeuchtung etc.) finden, weil Lippenstifte und Cremes von ihrer Rezeptur her unterschiedlich zusammengesetzt sind.

Es war daher die Aufgabe der vorliegenden Erfindung, eine kosmetische Pflegerezeptur zu entwickeln, die sich zu (physikalisch) unterschiedlichen Produkt-Typen (beispielsweise Stift und Creme) verarbeiten lässt.

Überraschend gelöst werden die Aufgaben durch eine kosmetische Wasser-in-ÖI-Emulsion (W/O-Emulsion) gemäß Anspruch 1.

Überraschend gelöst werden die Aufgaben ferner durch ein Verfahren zur Herstellung einer kosmetischen Wasser-in-Öl-Emulsion (W/O-Emulsion) gemäß Anspruch 2.

Im Rahmen der vorliegenden Offenbarung beziehen sich die Formulierungen "erfindungsgemäß", "erfindungsgemäße Zubereitung" etc. immer auf die erfindungsgemäßen Zubereitungen und Verfahren, d.h. auch auf Zubereitungen, mit denen das erfindungsgemäße Verfahren verwirklicht wird.

Zwar kennt der Stand der Technik die WO 2019/182198 A1, DE 102018217130 A1, US 2015/011654 A1, KR 2019/0057660 A sowie den Datenbank-Eintrag in der GNPD Datenbank von Mintel mit der Eintragungsnummer 6245877 "Soothing Lip Balm", doch konnten diese Offenbarungen nicht den Weg zur vorliegenden Erfindung weisen.

Es ist vorteilhaft im Sinne der vorliegenden Erfindung, wenn als erster Emulgator Diisostearoyl Polyglyceryl-3 Dimer Dilinoleate und als zweiter Emulgator Polyglyceryl-4 Diisostearate/Polyhydroxystearate/Sebacate eingesetzt wird.

Die erfindungsgemäßen Zubereitungen sind bevorzugt dadurch gekennzeichnet, dass die Gesamtkonzentration des (einer) oder der (beiden) ersten Emulgatoren von 1,2 bis 2,5 Gewichts-%, bezogen auf das Gesamtgewicht der Zubereitung beträgt.

Desweiteren sind die erfindungsgemäß vorteilhaften Ausführungsformen der vorliegenden Erfindung dadurch gekennzeichnet, dass die Gesamtkonzentration des oder der zweiten Emulgatoren 0,5 bis 2,5 Gewichts-%, bezogen auf das Gesamtgewicht der Zubereitung, beträgt. Erfindungsgemäß bevorzugt ist dabei eine Gesamtkonzentration von 0,8 bis 1,5 Gewichts-%, bezogen auf das Gesamtgewicht der Zubereitung.

Die erfindungsgemäß vorteilhafte Einsatzkonzentration für Sonnenblumenwachs beträgt 1 bis 10 Gewichts-%, bezogen auf das Gesamtgewicht der Zubereitung. Dabei ist eine Einsatzkonzentration von 1,5 bis 4,5 Gewichts-%, bezogen auf das Gesamtgewicht der Zubereitung, erfindungsgemäß bevorzugt.

Es ist darüber hinaus vorteilhaft im Sinne der vorliegenden Erfindung, wenn die Menge der Wasserphase 35 bis 65 Gewichts-% und die Menge der Ölphase 35 bis 65 Gewichts-%, bezogen auf die Gesamtmenge der Zubereitung, beträgt. Dabei soll die Gesamtsumme beider Phasen vorteilhaft 100 Gewichts-% betragen.

Erfindungsgemäß vorteilhafte Ausführungsformen der vorliegenden Erfindung werden ferner dadurch erhalten, dass die Zubereitung Ester enthält, dessen Alkohol- und Säure-Einheit jeweils Kohlenwasserstoffketten der Kettenlänge C14 bis C62 umfasst.

Dabei ist es erfindungsgemäß bevorzugt, wenn die Zubereitung Behenylbehenat (INCI: Behenyl Behenate) enthält.

Desweitern ist es erfindungsgemäß von Vorteil, wenn die erfindungsgemäße Zubereitung Bienenwachs (INCI: Cera alba) enthält. Die erfindungsgemäß vorteilhafte Einsatzkonzentration für das Bienenwachs beträgt von 1 bis 10 Gewichts-%, bezogen auf das Gesamtgewicht der Zubereitung.

Es war für den Fachmann insbesondere überraschend, dass für die Stabilisierung der W/O-Emulsion keine zusätzlichen Salze benötigt werden, wie es üblicherweise für W/O-Emulsionen der Fall ist. Vielmehr ist es vorteilhaft im Sinne der vorliegenden Erfindung, wenn der Salzgehalt der Zubereitung kleiner/gleich 0,1 Gewichts-%, bezogen auf das Gesamtgewicht der Zubereitung beträgt.

Darüber hinaus ist es vorteilhaft im Sinne der vorliegenden Erfindung, wenn die Zubereitung Glycerin enthält. In einem solchen Falle sind die erfindungsgemäß bevorzugten Ausführungsformen der vorliegenden Erfindung dadurch gekennzeichnet, dass die Zubereitung Glycerin in einer Menge von 1 bis 20 Gewichts-%, bezogen auf das Gesamtgewicht der Zubereitung, enthält.

Darüber hinaus ist es erfindungsgemäß vorteilhaft, wenn die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; Thiamidol; 8-Hexadecen-1,16-dicarbonsäure, Glycerylglycose, Allantoin, Vitamin E bzw. Vitamin-E Acetat, Hyaluronsäure und/oder deren Salze, Licochalcon A und/oder Pflanzenextrakte enthält.

Erfindungsgemäß bevorzugt ist dabei der Einsatz der Verbindungen Panthenol, Allantion und Hyaluronsäure.

Des Weiteren kann die erfindungsgemäße Emulsion entsprechend ihres Einsatzzweckes, die entsprechenden, üblichen kosmetischen Inhaltsstoffe und lipophilen Komponenten enthalten.

Zu den für den Fachmann nicht vorhersehbaren Besonderheiten der vorliegenden Erfindung gehört der Umstand, dass sich mit ein und derselben Rezeptur, je nach Herstellungsverfahren entweder fließfähige Cremes und Salben oder feste Stiftformulierungen (beispielsweise für Lippenstifte) herstellen lassen. Ist die erfindungsgemäße Emulsion erst einmal mittels Heiß-Heiß-Verfahren hergestellt, kann sie entsprechend der weiteren Verfahrensschritte in eine Creme/Lotion oder eine Stiftmasse weiterverarbeitet werden. Auch ist es möglich, bereits hergestellte Cremes/Lotionen/Salben oder Stifte durch einfaches Erhitzen unter Anwendung der weiteren Verfahrensschritte in die jeweils andere Produktkategorie zu überführen, wodurch sich neue, Ressourcen sparende Optionen bei der Verarbeitung von Produktionsüberschüssen ergeben.

Zur Herstellung einer Creme, Lotion oder Salbe wird dabei wie folgt vorgegangen:
Hier zeichnet sich das erfindungsgemäße Verfahren dadurch aus, dass nach der Homogenisierung bei einer Temperatur von größer/gleich 80 °C die Zubereitung unter Rühren auf 40 bis 45 °C abgekühlt und anschließend erneut homogenisiert wird.

In einem weiteren erfindungsgemäß vorteilhaften Verfahrensschritt wird die erfindungsgemäße Zubereitung nach der zweiten Homogenisierung weiter abgekühlt und bei einer Temperatur von 35 bis 40 °C in Vorratsbehältnisse abgefüllt.

Zur Herstellung einer stiftförmigen Formulierung wird hingegen wie folgt vorgegangen:
Hier zeichnet sich das erfindungsgemäße Verfahren dadurch aus, dass nach der Homogenisierung bei einer Temperatur von größer/gleich 80 °C die Zubereitung unter Rühren auf 70 bis 75 °C abgekühlt und anschließend in eine Gießform für Stifte vergossen und in dieser mit einer Abkühlgeschwindigkeit von 0,2 - 0.4°C/sek auf Raumtemperatur abgekühlt wird.

Die Abkühlrate nach dem Vergießen des Stiftes sollte also möglichst hoch sein.

Ein derartig hergestellter Stift besitzt auch bei 40°C noch ausreichende Formstabilität, so dass eine Applikation möglich ist, ohne dass der Stift abbricht oder zerdrückt wird.

Dieses Verfahren zur Stiftherstellung ist erfindungsgemäß vorteilhaft dadurch gekennzeichnet, dass nach dem Abkühlen auf Raumtemperatur die stiftförmige Masse (Stiftmine) entformt und in ein Vorratsbehältnis für stiftförmige Zubereitungen umgefüllt wird.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **INCI** | **Beispiel 1** | **Beispiel 2** | **Beispiel 3** | **Beispiel 4** |
|---|---|---|---|---|---|
| Phase A | Behenyl Behenate | 5 | 4 | | 4 |
| | Prunus Amygdalus Dulcis Oil | 20 | 20 | 20 | 20 |
| | Cera Alba | | | 4 | |
| | Ricinus Communis Seed Oil | 10 | 10 | 10 | 10 |
| | Helianthus Annuus Seed Cera | 1 | 2,5 | 2 | 2,5 |
| | Diisostearoyl Polyglyceryl-3 Dimer Dilinoleate | 1,4 | 1,4 | 1,4 | |
| | Polyglyceryl-4 Diisostearate/Polyhydroxystearate/Sebacate | 0,8 | 0,8 | 0,8 | |
| | Polyglyceryl-3 Polyricinoleate | | | | 1,4 |
| | Polyglyceryl-2 Sesquioleate | | | | 0,8 |
| Phase B | Phenoxyethanol | 0,8 | 0,8 | 0,8 | 0,8 |
| | Glyceryl Caprate | 0,3 | 0,3 | 0,3 | 0,3 |
| | Glycerin | 10 | 10 | 10 | 10 |
| | Caprylyl Glycol | 0,15 | 0,15 | 0,15 | 0,15 |
| | Aqua | 50,55 | 50,05 | 50,55 | 50,05 |
| | | 100 | 100 | 100 | 100 |

### - Herstellung feste Formulierung:

- Phase A unter Rühren auf 90°C erhitzen
- Phase B unter Rühren auf 85°C erhitzen
- Phase B langsam zu Phase A geben, Rühren mit 800 - 1200 rpm
- Emulsion bei 80°C 20 Minuten nachrühren
- Anschließend bei 70-75°C in die Gießform geben
- Bei Raumtemperatur 15 Minuten auskühlen lassen. Die Abkühlgeschwindigkeit beträgt hierbei ca. 0,3°C/sek. Anschließend weitere 30 Minuten bei -18°C aushärten lassen
- Empfohlenes Equipment: IKA Rührwerk mit passendem Dissolver

### - Herstellung Creme Formulierung:

- Phase A unter Rühren auf 90°C erhitzen
- Phase B unter Rühren auf 85°C erhitzen
- Phase B langsam zu Phase A geben, Rühren mit 150 rpm
- Heiße Emulsion homogenisieren
- Anschließend kalt Rühren (50-70 rpm)
- Emulsion bei 30°C nochmals homogenisieren

## Patentansprüche

1. Kosmetische Wasser-in-Öl-Emulsion (W/O-Emulsion) enthaltend
a) einen ersten Emulgator aus der Gruppe der Verbindungen Diisostearoyl Polyglyceryl-3 Dimer Dilinoleate und/oder Polyglyceryl-3 Polyricinoleate
b) einem zweiten Emulgator aus der Gruppe der Verbindungen Polyglyceryl-4 Diisostearate/Polyhydroxystearate/Sebacate und/oder Polyglyceryl-2 Sesquioleate
c) Sonnenblumenwachs, wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen), Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, **dadurch gekennzeichnet, dass** die Gesamtkonzentration des oder der ersten Emulgatoren a) 1 bis 4 Gewichts-%, bezogen auf das Gesamtgewicht der Zubereitung, beträgt.

2. Verfahren zur Herstellung einer kosmetischen Wasser-in-Öl-Emulsion (W/O-Emulsion), wobei die Ölphase
a) einen ersten Emulgator aus der Gruppe der Verbindungen Diisostearoyl Polyglyceryl-3 Dimer Dilinoleate und/oder Polyglyceryl-3 Polyricinoleate
b) einem zweiten Emulgator aus der Gruppe der Verbindungen Polyglyceryl-4 Diisostearate/Polyhydroxystearate/Sebacate und/oder Polyglyceryl-2 Sesquioleate und
c) Sonnenblumenwachs enthält, wobei die Zubereitung frei ist von Mineralöl, Paraffinwachs, mikrokristallinem Wachs, Schellackwachs und Polyethylenwachsen, frei von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen), Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern, wobei die Gesamtkonzentration des oder der ersten Emulgatoren 1 bis 4 Gewichts-%, bezogen auf das Gesamtgewicht der Zubereitung, beträgt, **dadurch gekennzeichnet, dass** die wässrige Phase und die Ölphase zunächst bei einer Temperatur von jeweils größer/gleich 80 °C vereinigt und homogenisiert werden.

3. W/O-Emulsion nach Anspruch 1 oder Verfahren nach Anspruch 2, **dadurch gekennzeichnet, dass** als erster Emulgator Diisostearoyl Polyglyceryl-3 Dimer Dilinoleate und als zweiter Emulgator Polyglyceryl-4 Diisostearate/Polyhydroxystearate/Sebacate eingesetzt wird.

4. W/O-Emulsion oder Verfahren nach einem der vorhergehenden Ansprüche, **dadurch gekennzeichnet, dass** die Gesamtkonzentration des oder der zweiten Emulgatoren 0,5 bis 2,5 Gewichts-%, bezogen auf das Gesamtgewicht der Zubereitung, beträgt.

5. W/O-Emulsion oder Verfahren nach einem der vorhergehenden Ansprüche, **dadurch gekennzeichnet, dass** die Konzentration an Sonnenblumenwachs 1 bis 10 Gewichts-%, bezogen auf das Gesamtgewicht der Zubereitung, beträgt.

6. W/O-Emulsion oder Verfahren nach einem der vorhergehenden Ansprüche, **dadurch gekennzeichnet, dass** die Menge der Wasserphase 35 bis 65 Gewichts-% und die Menge der Ölphase 35 bis 65 Gewichts-%, bezogen auf die Gesamtmenge der Zubereitung, beträgt.

7. W/O-Emulsion oder Verfahren nach einem der vorhergehenden Ansprüche, **dadurch gekennzeichnet, dass** die Zubereitung Ester enthält, dessen Alkohol- und Säure-Einheit jeweils Kohlenwasserstoffketten der Kettenlänge C14 bis C62 umfasst.

8. W/O-Emulsion oder Verfahren nach einem der vorhergehenden Ansprüche, **dadurch gekennzeichnet, dass** die Zubereitung Behenylbehenat (INCI: Behenyl Behenate) enthält.

9. W/O-Emulsion oder Verfahren nach einem der vorhergehenden Ansprüche, **dadurch gekennzeichnet, dass** die Zubereitung Bienenwachs (INCI: Cera alba) enthält.

10. W/O-Emulsion oder Verfahren nach einem der vorhergehenden Ansprüche, **dadurch gekennzeichnet, dass** der Salzgehalt der Zubereitung kleiner/gleich 0,1 Gewichts-%, bezogen auf das Gesamtgewicht der Zubereitung beträgt.

11. W/O-Emulsion oder Verfahren nach einem der vorhergehenden Ansprüche, **dadurch gekennzeichnet, dass** die Zubereitung Glycerin in einer Menge von 1 bis 20 Gewichts-%, bezogen auf das Gesamtgewicht der Zubereitung, enthält.

12. W/O-Emulsion oder Verfahren nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; Thiamidol; 8-Hexadecen-1,16-dicarbonsäure, Glycerylglycose, Allantoin, Vitamin E bzw. Vitamin-E Acetat, Hyaluronsäure und/oder deren Salze, Licochalcon A und/oder Pflanzenextrakte enthält.

13. Verfahren nach einem der vorhergehenden Ansprüche, **dadurch gekennzeichnet, dass** nach der Homogenisierung bei einer Temperatur von größer/gleich 80 °C die Zubereitung unter Rühren auf 40 bis 45 °C abgekühlt und anschließend erneut homogenisiert wird.

14. Verfahren nach Anspruch 13, **dadurch gekennzeichnet, dass** die Zubereitung nach der zweiten Homogenisierung weiter abgekühlt und bei einer Temperatur von 35 bis 40 °C in Vorratsbehältnisse abgefüllt wird.

15. Verfahren nach einem der Ansprüche 2 bis 12, **dadurch gekennzeichnet, dass** nach der Homogenisierung bei einer Temperatur von größer/gleich 80 °C die Zubereitung unter Rühren auf 70 bis 75 °C abgekühlt und anschließend in eine Gießform für Stifte vergossen und in dieser mit einer Abkühlungsrate von 0,2-0,4 °C/sek auf Raumtemperatur abgekühlt wird.

16. Verfahren nach Anspruch 15, **dadurch gekennzeichnet, dass** nach dem Abkühlen auf Raumtemperatur die stiftförmige Masse (Stiftmine) entformt und in ein Vorratsbehältnis für stiftförmige Zubereitungen umgefüllt wird.

## Claims

1. Cosmetic water-in-oil emulsion (W/O emulsion) comprising
a) a first emulsifier from the group of compounds comprising diisostearoyl polyglyceryl-3 dimer dilinoleate and/or polyglyceryl-3 polyricinoleate
b) a second emulsifier from the group of compounds comprising polyglyceryl-4 diisostearate/polyhydroxystearate/sebacate and/or polyglyceryl-2 sesquioleate
c) sunflower wax, wherein the preparation is free from mineral oil, paraffin wax, microcrystalline wax, shellac wax and polyethylene waxes, free from polyacrylates, acrylate/C10-C30 alkyl acrylate crosspolymers and vinylpyrrolidone/hexadecene copolymers, and free from 3-(4-methylbenzylidene)camphor, 2-hydroxy-4-methoxybenzophenone (INCI: Oxybenzone), 2-ethylhexyl 4-methoxycinnamate (INCI Octyl Methoxycinnamate), ethylhexyl 2-cyano-3,3-diphenylacrylate (INCI: Octocrylene, parabens (particularly methyl, propyl and butyl paraben), methylisothiazolinone, chloromethylisothiazolinone and DMDM hydantoin, polyethylene glycol ethers or polyethylene glycol esters, **characterized in that** the total concentration of the first emulsifier(s) a) is 1 to 4% by weight, based on the total weight of the preparation.

2. Process for producing a cosmetic water-in-oil emulsion (W/O emulsion), wherein the oil phase comprises
a) a first emulsifier from the group of compounds comprising diisostearoyl polyglyceryl-3 dimer dilinoleate and/or polyglyceryl-3 polyricinoleate
b) a second emulsifier from the group of compounds comprising polyglyceryl-4 diisostearate/polyhydroxystearate/sebacate and/or polyglyceryl-2 sesquioleate and
c) sunflower wax, wherein the preparation is free from mineral oil, paraffin wax, microcrystalline wax, shellac wax and polyethylene waxes, free from polyacrylates, acrylate/C10-C30 alkyl acrylate crosspolymers and vinylpyrrolidone/hexadecene copolymers, and free from 3-(4-methylbenzylidene)camphor, 2-hydroxy-4-methoxybenzophenone (INCI: Oxybenzone), 2-ethylhexyl 4-methoxycinnamate (INCI Octyl Methoxycinnamate), ethylhexyl 2-cyano-3,3-diphenylacrylate (INCI: Octocrylene, parabens (in particular methyl, propyl and butyl paraben), methylisothiazolinone, chloromethylisothiazolinone and DMDM hydantoin, polyethylene glycol ethers or polyethylene glycol esters, where the total concentration of the first emulsifier(s) is 1 to 4% by weight, based on the total weight of the preparation, **characterized in that** the aqueous phase and the oil phase are first combined and homogenized at a temperature equal to/greater than 80°C in each case.

3. W/O emulsion according to Claim 1 or process according to Claim 2, **characterized in that** diisostearoyl polyglyceryl-3 dimer dilinoleate is used as first emulsifier and polyglyceryl-4 diisostearate/polyhydroxystearate/sebacate is used as second emulsifier.

4. W/O emulsion or process according to any of the preceding claims, **characterized in that** the total concentration of the second emulsifier(s) is 0.5 to 2.5% by weight, based on the total weight of the preparation.

5. W/O emulsion or process according to any of the preceding claims, **characterized in that** the concentration of sunflower wax is 1 to 10% by weight, based on the total weight of the preparation.

6. W/O emulsion or process according to any of the preceding claims, **characterized in that** the amount of the water phase is 35 to 65% by weight and the amount of the oil phase is 35 to 65% by weight, based on the total amount of the preparation.

7. W/O emulsion or process according to any of the preceding claims, **characterized in that** the preparation comprises esters, of which the alcohol and acid unit each comprises hydrocarbon chains of chain length C14 to C62.

8. W/O emulsion or process according to any of the preceding claims, **characterized in that** the preparation comprises behenyl behenate (INCI: Behenyl Behenate).

9. W/O emulsion or process according to any of the preceding claims, **characterized in that** the preparation comprises beeswax (INCI: Cera Alba).

10. W/O emulsion or process according to any of the preceding claims, **characterized in that** the salt content of the preparation is less than/equal to 0.1% by weight, based on the total weight of the preparation.

11. W/O emulsion or process according to any of the preceding claims, **characterized in that** the preparation comprises glycerin in an amount of 1 to 20% by weight, based on the total weight of the preparation.

12. W/O emulsion or process according to any of the preceding claims, **characterized in that** the preparation comprises one or more compounds selected from the group of compounds comprising alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, tocopheryl acetate, dihydroxyacetone; thiamidol, 8-hexadecene-1,16-dicarboxylic acid, glycerylglucose, allantoin, vitamin E and vitamin E acetate, hyaluronic acid and/or salts thereof, licochalcone A and/or plant extracts.

13. Process according to any of the preceding claims, **characterized in that** after the homogenization at a temperature of greater than/equal to 80°C, the preparation is cooled to 40 to 45°C while stirring and is then homogenized again.

14. Process according to Claim 13, **characterized in that** the preparation is cooled further after the second homogenization and is filled into storage containers at a temperature of 35 to 40°C.

15. Process according to any of Claims 2 to 12, **characterized in that** after the homogenization at a temperature of greater than/equal to 80°C, the preparation is cooled to 70 to 75°C while stirring and then poured into a casting mold for sticks and cooled therein to room temperature at a cooling rate of 0.2-0.4°C/second.

16. Process according to Claim 15, **characterized in that** after cooling to room temperature the stick-shaped mass (stick pencil) is removed from the mold and transferred to a storage container for stick-shaped preparations.

## Revendications

1. Émulsion cosmétique eau-dans-huile (émulsion E/H) contenant
a) un premier émulsifiant du groupe des composés diisostéaroyl polyglycéryl-3 dimère dilinoléate et/ou polyglycéryl-3 polyricinoléate
b) un deuxième émulsifiant du groupe des composés polyglycéryl-4 diisostéarate/polyhydroxystéarate/sébacate et/ou polyglycéryl-2 sesquioléate
c) de la cire de tournesol, la préparation étant exempte d'huile minérale, de cire de paraffine, de cire microcristalline, de cire de gomme laque et de cires de polyéthylène, exempte de polyacrylates, de polymères réticulés d'acrylate/acrylate d'alkyle en C10-C30 et de copolymères de vinylpyrrolidone/hexadécène, ainsi qu'exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), d'acrylate d'éthylhexyl-2-cyano-3,3-diphényle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol, **caractérisée en ce que** la concentration totale du ou des premiers émulsifiants a) est de 1 à 4 % en poids par rapport au poids total de la préparation.

2. Procédé de préparation d'une émulsion cosmétique eau dans huile (émulsion E/H), dans lequel la phase huileuse contient
a) un premier émulsifiant du groupe des composés diisostéaroyl polyglycéryl-3 dimère dilinoléate et/ou polyglycéryl-3 polyricinoléate
b) un deuxième émulsifiant du groupe des composés polyglycéryl-4 diisostéarate/polyhydroxystéarate/sébacate et/ou polyglycéryl-2 sesquioléate et
c) de la cire de tournesol, la préparation étant exempte d'huile minérale, de cire de paraffine, de cire microcristalline, de cire de gomme laque et de cires de polyéthylène, exempte de polyacrylates, de polymères réticulés d'acrylate/acrylate d'alkyle en C10-C30 et de copolymères de vinylpyrrolidone/hexadécène, ainsi qu'exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), d'acrylate d'éthylhexyl-2-cyano-3,3-diphényle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol, la concentration totale du ou des premiers émulsifiants étant de 1 à 4 % en poids par rapport au poids total de la préparation, **caractérisé en ce que** la phase aqueuse et la phase huileuse sont d'abord combinées et homogénéisées à une température supérieure ou égale à 80 °C.

3. Émulsion E/H selon la revendication 1 ou procédé selon la revendication 2, caractérisé(e) en ce que l'on utilise comme premier émulsifiant du diisostéaroyl polyglycéryl-3 dimère dilinoléate et comme deuxième émulsifiant du polyglycéryl-4 diisostéarate/polyhydroxystéarate/sébacate.

4. Émulsion E/H ou procédé selon l'une quelconque des revendications précédentes, caractérisé(e) en ce que la concentration totale du ou des deuxièmes émulsifiants est de 0,5 à 2,5 % en poids par rapport au poids total de la préparation.

5. Émulsion E/H ou procédé selon l'une quelconque des revendications précédentes, caractérisé(e) en ce que la concentration en cire de tournesol est de 1 à 10 % en poids, par rapport au poids total de la préparation.

6. Émulsion E/H ou procédé selon l'une quelconque des revendications précédentes, caractérisé(e) en ce que la quantité de phase aqueuse est de 35 à 65 % en poids et la quantité de phase huileuse est de 35 à 65 % en poids, par rapport à la quantité totale de la préparation.

7. Émulsion E/O ou procédé selon l'une quelconque des revendications précédentes, caractérisé(e) en ce que la préparation contient des esters dont les unités alcool et acide comprennent chacune des chaînes hydrocarbonées d'une longueur de chaîne C14 à C62.

8. Émulsion E/H ou procédé selon l'une quelconque des revendications précédentes, caractérisé(e) en ce que la préparation contient du béhénate de béhényle (INCI : Behenyl Behenate).

9. Émulsion E/O ou procédé selon l'une quelconque des revendications précédentes, caractérisé(e) en ce que la préparation contient de la cire d'abeille (INCI : Cera alba).

10. Émulsion E/H ou procédé selon l'une quelconue des revendications précédentes, caractérisé(e) en ce que la teneur en sel de la préparation est inférieure ou égale à 0,1 % en poids par rapport au poids total de la préparation.

11. Émulsion E/H ou procédé selon l'une quelconque des revendications précédentes, caractérisé(e) en ce que la préparation contient de la glycérine en une quantité de 1 à 20 % en poids par rapport au poids total de la préparation.

12. Émulsion E/H ou procédé selon l'une quelconque des revendications précédentes, caractérisé(e) en ce que la préparation contient un ou plusieurs composés choisis dans le groupe des composés acide alpha-liponique, acide folique, phytoène, D-biotine, coenzyme Q10, alpha-glucosylrutine, carnitine, carnosine, isoflavonoïdes naturels et/ou synthétiques, flavonoïdes, créatine, créatinine, taurine, β-alanine, panthénol, magnolol, honokiol, acétate de tocophéryle, dihydroxyacétone ; thiamidol ; acide 8-hexadécène-1,16-dicarboxylique, glycérylglucose, allantoïne, vitamine E ou acétate de vitane E, acide hyaluronique et/ou ses sels, licochalcone A et/ou extraits végétaux.

13. Procédé selon l'une quelconque des revendications précédentes, **caractérisé en ce qu'**après l'homogénéisation à une température supérieure ou égale à 80 °C, la préparation est refroidie à 40 à 45 °C sous agitation, puis homogénéisée à nouveau.

14. Procédé selon la revendication 13, **caractérisé en ce que** la préparation est encore refroidie après la deuxième homogénéisation et versée dans des récipients de stockage à une température de 35 à 40 °C.

15. Procédé selon l'une quelconque des revendications 2 à 12, **caractérisé en ce qu'**après l'homogénéisation à une température supérieure ou égale à 80 °C, la préparation est refroidie à 70 à 75 °C sous agitation, puis coulée dans un moule pour crayons et refroidie dans celui-ci à une vitesse de refroidissement de 0,2 à 0,4 °C/s jusqu'à température ambiante.

16. Procédé selon la revendication 15, **caractérisé en ce qu'**après refroidissement à température ambiante, la masse en forme de crayon (mine de crayon) est démoulée et transférée dans un récipient de stockage pour préparations en forme de crayons.
